(19) Europäisches Patentamt
European Patent Office
Office européen des brevets

(11) Publication number: **0 151 702 B1**

(12) # EUROPEAN PATENT SPECIFICATION

(45) Date of publication of patent specification: **25.11.87**

(51) Int. Cl.$^4$: **C 07 C 43/315,** C 07 D 209/46, C 07 D 333/22

(21) Application number: **84113585.8**

(22) Date of filing: **10.09.81**

(60) Publication number of the earlier application in accordance with Art. 76 EPC: **0 048 136**

(54) **Alpha-hydroxyketone acetals.**

(30) Priority: **11.09.80 JP 125355/80**
**15.10.80 JP 143042/80**
**10.11.80 JP 157049/80**
**30.01.81 JP 11700/81**
**15.06.81 JP 90979/81**

(43) Date of publication of application:
**21.08.85 Bulletin 85/34**

(45) Publication of the grant of the patent:
**25.11.87 Bulletin 87/48**

(84) Designated Contracting States:
**AT BE CH DE FR GB IT LI LU NL SE**

(56) References cited:

**THE JOURNAL OF ORGANIC CHEMISTRY, vol. 45, no. 14, 4th July 1980, pages 2803-2813; N.DeKIMPE et al.: "Rearrangement of 1-aryl-2,2-dihalo-1-alkanones"**

(73) Proprietor: **SYNTEX PHARMACEUTICALS INTERNATIONAL LIMITED**
**Corner House Church Street**
**Hamilton 5 (BM)**

(72) Inventor: **Tsuchihashi, Genichi**
**2-14-7 Sakuragaoka**
**Tama-shi Tokyo (JP)**
Inventor: **Mitamura, Shuichi**
**1-9-2 Minamidai**
**Sagamihara-shi Kanagawa-ken (JP)**
Inventor: **Kitajima, Kouji**
**1-9-1-203 Minamidai**
**Sagamihara-shi Kanagawa-ken (JP)**

(74) Representative: **Myerscough, Philip Boyd et al**
**J.A.Kemp & Co. 14, South Square Gray's Inn**
**London, WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 151 702 B1

(56) References cited:

JOURNAL OF HETEROCYCLIC CHEMISTRY, vol. 6, no. 6, December 1969, pages 891-900; A.T.NIELSEN et al.: "The hydroxyacetylpiperidines and their N-benzyl derivatives. The 2-hydroxymethyl-2-piperidyl- and 2-hydroxymethyl-2-pyridyl-1,3-dioxolanes"

TETRAHEDRON LETTERS, no. 43, pages 4155-4158, Pergamon Press Ltd., GB; A.I.MEYERS et al.: "2-aryl acetals as acyl anion equivalents"

CHEMICAL ABSTRACTS, vol. 90, no. 25, 18th June 1979, page 617, no. 203985f, Columbus, Ohio, US; H.NAKAMURA et al.: "Synthesis of 2-(1-methyl-3-indolyl)-3-alkyl or aryl-1,4-dioxenes"

TETRAHEDRON LETTERS, vol. 22, no. 14, 1981, pages 1283-1286, Pergamon Press Ltd., GB; R.M.MORIARTY et al.: "Direct alpha-hydroxylation of ketones using iodosobenzene"

**Description**

This invention relates to novel alpha-hydroxyketone acetals which can be used to prepare alpha-aromatic group substituted alkanoic acids or esters of the general formula

$$Ar\text{-}\overset{\overset{R^1}{|}}{C}H\text{—}COOR^2 \qquad (I)$$

wherein Ar represents an aromatic group, $R^1$ represents a hydrogen atom or a saturated aliphatic group, or Ar and $R^1$ may form a condensed ring together with the carbon atom to which they are bonded, and $R^2$ represents a hydrogen atom, an alkyl group or a hydroxyalkyl group.

The preparation of the substituted alkanoic acids and esters (I) is described in EP—A—48136, from which this application is divided.

J. Org. Chem. 1960, *45,* 2803—2813 "Rearrangement of 1-aryl-2,2-dihalo-1-alkanones" describes 1,1-dimethoxy-1-phenyl-2-propanol as an example of a compound produced by an $S_N2$ reaction.

Journal of Heterocyclic Chemistry, December 1969, *6,* 891—900 "The hydroxy-acetylpiperidines and their N-benzyl derivatives" describes 2-hydroxymethyl-2-pyridyl-1,3-dioxolanes and 2-hydroxymethyl-2-(N-benzylpiperidine)-1,3-dioxolanes and for use as intermediates for preparing, inter alia, piperidyl-1,2-ethanediols.

Tetrahedron letters, 1979, *43,* 4155—4158 "2-aryl acetals as acyl anion equivalents" describes 2-(1-hydroxy-2-methylpropyl)-2-(p-oxazolinylphenyl)-1,3-dioxolane.

Heterocycles 1978, *10,* 167—70 (Chemical Abstracts 90:2039851) "Synthesis of 2-(1-methyl-3-indolyl)-3-alkyl or aryl-1,4-dioxenes" describes 2-(1-methyl-3-indolyl)-2-hydroxymethyl-1,3-dioxolane and its derivative wherein the hydroxymethyl group is substituted with a methyl group.

None of these documents discloses that compounds of the present invention could be useful intermediates in preparing compound of general formula (I).

Many of the compounds of general formula (I) are commercially valuable. For example, alpha-(4-isobutylphenyl)propionic acid corresponding to a compound of general formula (I) in which Ar is a 4-isobutylphenyl group, $R^1$ is a methyl group, and $R^2$ is a hydrogen atom, is known as "Ibuprofen" which is an anti-inflammatory drug.

Alpha-(6-methoxy-2-naphthyl)propionic acid corresponding to a compound of general formula (I) in which Ar is a 6-methoxy-2-naphthyl group, $R^1$ is a methyl group, and $R^2$ is a hydrogen atom is known as "Naproxen". Alpha-(4-difluoromethoxyphenyl)isovaleric acid, corresponding to a compound of general formula (I) in which, Ar is a 4-difluoromethoxyphenyl group, $R^1$ is an isopropyl group, and $R^2$ is a hydrogen atom is very effective as an acid moiety of pyrethroid insecticides.

Many processes have been described for the production of alpha-aromatic group substituted alkanoic acids. In EP—A—48136 we describe and claim a process for preparing alpha-aromatic group substituted alkanoic acids and esters of the general formula (I) by hydrolyzing, or by teating with an agent having affinity for oxygen, an alpha-sulfonyloxyketone acetal of the general formula

$$Ar\text{-}\underset{\underset{OR^4}{|}}{\overset{\overset{OR^3}{|}}{C}}\text{ — }\overset{\overset{OSO_2\text{—}R^5}{|}}{C}H\text{—}R^1 \qquad (II)$$

wherein $R^3$ and $R^4$, independently represent alkyl groups or together represent an alkylene group, $R^5$ represents a substituted or unsubstituted alkyl group or an aromatic group, and Ar and $R^1$ are as defined above. The alpha-sulfonyloxyketone acetals of formula (II) are also described and claimed in EP—A—48136.

The present invention provides alpha-hydroxyketone acetals which may readily be converted into the alpha-sulfonyloxyketone acetals of formula (II) and thence into the alkanoic acids and esters of formula (I).

According to the invention there are provided alpha-hydroxyketone acetals of the formula (VI):

$$Ar\text{-}\underset{\underset{OR^4}{|}}{\overset{\overset{OR^3}{|}}{C}}\text{ — }\overset{\overset{OH}{|}}{C}H\text{—}R^1 \qquad (VI)$$

wherein Ar represents an aromatic group, $R^1$ represents a hydrogen atom or a saturated aliphatic group, or Ar and $R^1$ form a condensed ring together with the carbon atom to which they are bonded, and $R^3$ and $R^4$ independently represent alkyl groups or together represent an alkylene group, subject to the provisos that:

(i) Ar does not represent phenyl or 4-chlorophenyl when $R^1$ is a hydrogen atom;

(ii) Ar does not represent phneyl when all of $R^1$, $R^3$ and $R^4$ are methyl;

(iii) Ar does not represent an unsubstituted pyridyl group when $R^3$ and $R^4$ together represent an ethylene group and $R^1$ represents a hydrogen atom;

(iv) Ar does not represent a p-(4,4-dimethyl-oxoazolin-2-yl-phenyl group when $R^3$ and $R^4$ together represent an ethylene group and $R^1$ represents an isopropyl group; and

(v) Ar does not represent 1-methyl-3-indolyl when $R^3$ and $R^4$ together represent an ethylene group and $R^1$ represents a hydrogen atom or a methyl group.

Preferred is a group of compounds of the formula (VI) wherein Ar represents a 6-methoxy-2-naphthyl group, a 4-isobutylphenyl group, a 4-lower alkoxyphenyl group, a 4-difluoromethoxyphenyl group, a 2-thienyl group, a 4-(1-oxo-2-isoindolinyl)phenyl group, a 4-biphenylyl group, or a 4-(tert-butyl)phenyl group.

The term "aromatic group", as used herein, is to be taken in its broadest meaning, and denotes a group of a cyclic compound having aromaticity. The aromaticity means a phenomenon wherein the ring is stabilized by the delocalization of π electrons. Generally, a ring having (4n+2) conjugated π electrons is stable and exhibits aromaticity. Thus, the aromatic group, as used herein, refers to a group of compounds having (4n+2) conjugated π electrons in the main ring. It can be classified roughly into aryl groups optionally having at least one substituent and heretoaromatic groups optionally having at least one substituent, which are described in detail below.

(a) Aryl groups optionally having at least one substituent.

The aryl groups are aromatic hydrocarbon groups of the monocyclic, polycyclic or condensed polycyclic type, and include, for example, phenyl, biphenyl and naphthyl.

The aryl groups may be unsubstituted, or have one or more substituents on the aromatic ring. Specific examples of the substituents include halogen atoms such as chlorine, bromine, fluorine, and iodine; lower alkyl groups such as methyl, ethyl, n-propyl, isopropyl, n-butyl, isobutyl, and tert-butyl; cycloalkyl groups such as cyclohexyl and cyclopentyl; aralkyl groups such as benzyl; lower alkoxy groups such as methoxy; ethoxy, n-propoxy and isopropoxy; lower alkylthio groups such as methylthio, ethylthio, n-propylthio, isopropylthio, and butylthio; arylthio groups such as phenylthio, tolylthio, and naphthylthio; alkenylthio groups such as alkylthio; aralkylthio such as benzylthio; acyloxy groups such as acetoxy; aroyloxy groups such as benzoyloxy; silyloxy groups such as trimethylsilyloxy; lower alkenyl groups such as allyl and prenyl $[(CH_3)_2C{=}CH{-}CH_2{-}]$; lower alkenyloxy groups such as allyloxy; aralkyloxy groups such as benzyloxy and phenethyloxy; aryloxy groups such as phenoxy; lower haloalkyl groups such as trifluoroethyl and trifluoropropyl; lower haloalkoxy groups such as difluoromethoxy and trifluoromethoxy; 4- to 6-membered heterocyclic groups such as indolinyl, oxo-isoindolynyl thienyl, piperidino and phthalimido; 4- to 6-membered heterocycloxy groups such as thiazoyloxy, and pyridyloxy; a nitro group; aroyl groups such as benzoyl; acylamino groups such as acetylamino and propionylamino; aroylamino groups such as benzoylamino; and dialkylamino groups such as dimethylamino and dibenzylamino. When these substituents are present, 1 to 5, preferably 1 to 3, of them are preferably present on the aromatic ring.

Specific examples of aryl groups having such substituents on the aromatic ring include chlorophenyl, fluorophenyl, bromophenyl, iodophenyl, tolyl, ethylphenyl, isopropylphenyl, isobutylphenyl, tert-butylphenyl, cyclohexylphenyl, methoxyphenyl, ethoxyphenyl, isopropoxyphenyl, methylthiophenyl, ethylthiophenyl, n-propylthiophenyl, isopropylthiophenyl, butylthiophenyl, phenylthiophenyl, tolylthiophenyl, allylthiophenyl, benzylthiophenyl, acetoxyphenyl, trimethylsilyloxyphenyl, benzoyloxyphenyl, benzylphenyl, prenylphenyl, allyloxyphenyl, benzyloxyphenyl phenoxyphenyl, tetrafluoroethoxyphenyl, trifluoroethylphenyl, trifluoromethoxyphenyl, difluoromethoxyphenyl, oxo-isoindolinylphenyl, thioazolyloxyphenyl, nitrophenyl, benzoylphenyl, acetylaminophenyl, piperidinophenyl, fluorobiphenyl, acetylaminobiphenyl, and methoxynaphthyl, methylthienylphenyl, acetylamino-chloro-phenyl-chloro-cyclohexyl-phenyl.

(b) Heteroaromatic groups optionally having at least one substitutent.

The heteroaromatic group may be of any of the monocyclic or condenssed polycyclic type. The heteroatom of the heteroaromatic ring may be nitrogen, oxygen or sulfur. The heteroaromatic ring contains generally 1 to 4, preferably 1 to 3, such hetero atoms, and may be generally 5- to 14- membered, preferably 5- to 9-membered. Specific examples of such heteroaromatic groups are thienyl, furyl, pyrrolyl, indolyl, phenothiazinyl, pyridyl, thiazolyl, and benzothiazolyl.

The heteroaromatic group may be unsubstituted, or may contain one or more substituents on the ring. Examples of substituents which may be present on the heterocyclic aromatic ring are lower alkyl groups such as methyl, ethyl, propyl and butyl; aryl groups such as phenyl and fluorophenyl; halogen atoms such as chlorine and fluorine; lower alkoxy groups such as methoxy, ethoxy and propoxy; aryloxy groups such as a phenoxy lower alkyl groups such as methyl, ethyl, propyl, and butyl; aralkyl groups such as benzyl; aryl groups such as phenyl, tolyl, and fluorophenyl; halogen atoms such as fluorine, chlorine and bromine; lower alkoxy groups such as methoxy, ethoxy, and propoxy; cycloalkyl groups such as cyclohexyl and cyclopentyl; lower alkenyl groups such as allyl and prenyl; lowr alkenyloxy groups such as allyloxy; aralkyloxy groups such as benzyloxy; aryloxy groups such as phenoxy; lower haloalkyl groups such as trifluoromethyl; lower alkylthio groups such as methylthio; arylthio groups such as phenylthio; aroyl

groups such as benzoyl, toluoyl, and chlorobenzoyl; acyl groups such as acetyl; lower haloalkyl groups such as trifluoromethyl; and cycloalkyl groups such as cyclohexyl.

When these substituents are present, 1 to 8, preferably 1 to 3, of them are desirably present.

Thus, examples of heteroaromatic groups having such substituents include methylphenothiazinyl, methoxymethyl-phenothiazinyl, methylpyrrolyl, toluoyl-methylpyrrolyl, phenylthienyl, bromothienyl, trifluoromethylthienyl, benzoylthienyl, cyclohexylthienyl, phenoxythienyl, methyl-methoxy-indolyl, chlorobenzoyl-indolyl, acetyl-pyrrolyl, methyl-toluoyl-pyrrolyl, benzopyrrolyl.

The term "saturated aliphatic group", as used in the present specification and the appended claims, denotes a linear, branched, or cyclic saturated aliphatic hydrocarbon group which may generally contain 1 to 10 carbon atoms, preferably 1 to 6 carbon atoms. Examples of such saturated aliphatic groups include linear or branched alkyl groups having 1 to 6 carbon atoms, especially lower alkyl groups, such as methyl, ethyl, n-propyl, isopropyl, n-butyl, isobutyl, tert-butyl, n-pentyl, isoamyl, and n-hexyl, and cycloalkyl groups having 3 to 10 carbon atoms, especially cycloalkyl groups having 3 to 7 carbon atoms, such as cyclopropyl, cyclopentyl, cyclohexyl, cycloheptyl.

Examples of the substituents which may be present on the alkyl group in the "substituted alkyl group" are halogen atoms such as chlorine, bromine and fluorine; aryl groups such as phenyl; alkoxy groups such as methoxy and ethoxy; and alicyclic groups such as [1R, 4R] or [1S, 4S]-7,7-dimethyl-2-oxobicyclo[2,2,1]heptan-1-yl. Specific examples of such substituted alkyl groups include trifluoromethyl, d- or l-10-camphoryl, and benzyl.

The "alkyl group" and "alkylene group", used herein, may be in any one of linear or branched types, and the alkylene groups are preferably lower alkylene groups such as ethylene, propylene, butylene, trimethylene and tetramethylene.

The term "lower", as used herein to qualify a group or a compound means that the group or compound so qualified has not more than 6, preferably not more than 4, carbon atoms.

The various groups in formula (VI) are exemplified as follows:

(1) Aryl groups optionally having at least substituent are preferred as the aromatic group Ar. More preferred are groups of the formula $R^6$-$Ar^1$-, wherein $Ar^1$ represents a phenylene or naphthylene group and $R^6$ represents a hydrogen atom, a halogen atom, a lower alkyl group, a lower alkoxy group, a lower haloalkoxy group, a lower alkanoylamino group, an oxo-isoindolinyl group, or a phenyl group. A thienyl group can be cited as another preferred example of the aromatic group Ar.

(2) Lower alkyl groups such as methyl, ethyl, n-propyl, isopropyl, or butyl are preferred as the saturated aliphatic group $R^1$. Thus, a hydrogen atom and lower alkyl groups constitute a preferred class of $R^1$.

(3) Lower alkyl groups are suitable as the alkyl groups represented individually by $R^3$ and $R^4$. Lower alkylene groups such as ethylene, propylene, and trimethylene are suitable as the alkylene group formed by $R^3$ and $R^4$ taken together.

(4) Examples of preferred substituted or unsubstituted alkyl groups represented by $R^5$ include unsubstituted lower alkyl groups, such as methyl, ethyl and butyl; lower haloalkyl groups such as trifluoromethyl; and a d- or l-10-camphoryl. Preferred as the aromatic group $R^5$ are substituted or unsubstituted phenyl groups of the formula

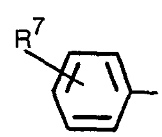

wherein $R^7$ represents a hydrogen atom, a halogen atom, a nitro group, or a lower alkyl group.

The α-hydroxyketone acetals of formula (VI) can be synthesized from alpha-haloketones of the general formula

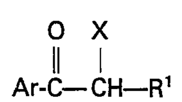

(V)

wherein X represents a halogen atom, and Ar and $R^1$ are as defined above, and then converted into the α-sulfonyloxyketone acetals of formula (II) through the route shown in the following reaction scheme.

Reaction Scheme

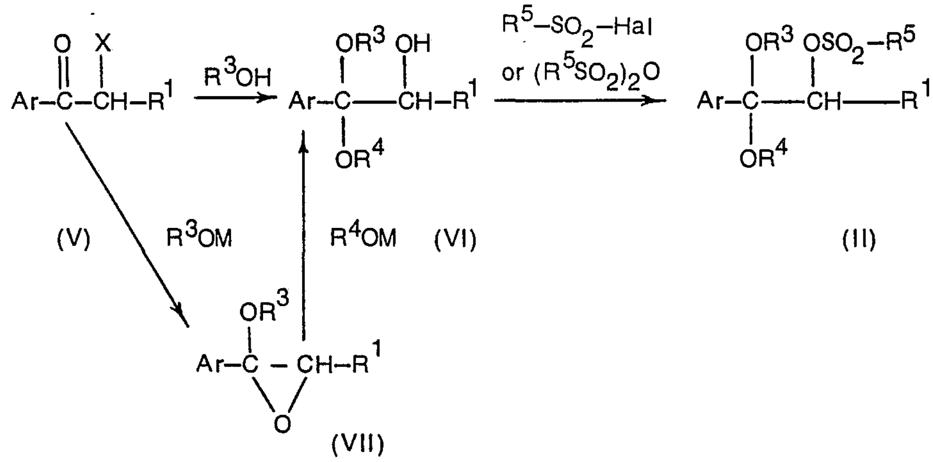


In the above scheme, M represents an alkali metal and Hal represents a halogen atom, especially a chlorine atom, and Ar, $R^1$, $R^3$, $R^4$, $R^5$ and X are as defined hereinabove.

The compound of formula (V) can be produced easily by subjecting a compound of the formula

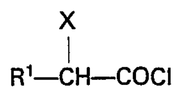

(VIII)

wherein $R^1$ and X are as defined above, to the Friedel-Crafts reaction with a compound of the formula

Ar-H (IX)

wherein Ar is as defined above, or by halogenating a compound of the formula

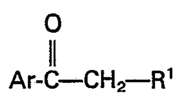

(X)

wherein Ar and $R^1$ are as defined above, in a manner known *per se.*

The route in the Reaction Scheme is described in detail below.

*Step (V)→(VI) (preparation of compounds of the present invention)*

This step involves the action of an alkali metal alkoxide ($R^3OM$) on the compound of general formula (V) in the presence of the corresponding alcohol ($R^3OH$) to give an alpha-hydroxyketone acetal of general formula (VI). In the compound of general formula (VI) produced in this step, $R^4$ is the same as $R^3$. Lithium alkoxides, sodium alkoxides and potassium alkoxides can be suitably used as the alkali metal alkoxide. The use of the sodium alkoxides is preferred because of their low cost. The amount of the alkali metal alkoxide is generally at least 1 mole per mole of the compound of formula (V), and the reaction can be completed rapidly if it is used in an amount of 1.5 to 3 moles per mole of the compound of formula (V). The amount of alcohol to be copresent may be at least 1 mole per mole of the compound of general formula (V). Advantageously, the alcohol is used in excess to make it serve also as a solvent. It is also possible to add an aprotic solvent which does not participate in the reaction, such as diethyl ether, tetrahydrofuran, DMF, or 1,2-di-methoxyethane. The reaction proceeds smoothly at a temperature of about −20°C to about 100°C. For the simplicity of the operation, the reaction is preferably carried out at room temperature to 60°C.

According to another embodiment, this step can be performed by reacting the compound of formula (V) with the alkali metal alkoxide ($R^3OM$) in an aprotic solvent such as diethyl ether, tetrahydrofuran or 1,2-dimethoxyethane to form an epoxy compound of formula (VIII), and then reacting it with, an alcohol ($R^4OH$) in the presence of a catalytic amount of an alkali metal alkoxide ($R^4OM$) to obtain an alpha-hydroxyketone acetal (VI) (see Examples 3 and 4). According to this process, a compound of formula (VI) in which $R^3$ differs from $R^4$ can also be produced.

The reaction between the compound of formula (V) and the alkali metal alkoxide ($R^3OM$) can be carried out usually at a temperature of about 0°C to about 60°C using 1 to 3 moles, per mole of the compound of formula (V), of the alkali metal alkoxide. The reaction between the compound of formula (VII) and the alcohol ($R^4OH$) can be performed generally at a temperature of about 0°C to about 100°C by using at least one mole, per mole of the compound of formula (VII), of the alcohol. Preferably, the alcohol is used in excess to make it serve also as a solvent.

According to still another embodiment of this step, the alpha-haloketone of general formula (V) is reacted in the presence of a dihydric alcohol such as ethylene glycol, propylene glycol or 1,3-propanediol with a mono-salt of the dihydric alcohol and an alkali metal to give the alpha-hydroxyketone acetal of general formula (VI). This process gives a product corresponding to formula (VI) in which $R^3$ and $R^4$ together form an alkylene group such as ethylene, propylene, or trimethylene (see Example 22).

The reaction can be performed generally at 0°C to 100°C by using at least 1 mole, preferably 1.5 to 3 moles, of the dihydric alcohol mono-salt of alkali metal in the presence of at least 0.5 mole, preferably 2 to 10 moles, of the dihydric alcohol. There can be added an aprotic solvent which does not participate in the reaction such as diethyl ether, tetrahydrofuran or 1,2-dimethoxyethane.

Typical examples of such compounds (VI) are given below in addition to those shown in the Examples. The compound (VI) wherein Ar is 4-prenylphenyl, $R^1$ is methyl; Ar is N-methylphenothiazinyl, $R^1$ is hydrogen or methyl; Ar is 1-methylpyrrolyl, $R^1$ is hydrogen or methyl; Ar is 2-fluoro-4-biphenylyl, $R^1$ is methyl; Ar is 2-acetyl-amino-4-biphenylyl, $R^1$ is methyl; Ar is 4-chlorophenyl, $R^1$ is isopropyl; and Ar is 3-chloro-4-(3-pyrrolin-1-yl)-phenyl, $R^1$ is methyl.

*Step (VI)→(II)*

This step involves the action of an O-sulfonylating agent of the formula $R^5$—$SO_2$—Hal or $(R^5SO_2)_2O$ on the alpha-hydroxyketone acetal of formula (VI) obtained in the above step to form the compound of general formula (II).

The following Examples illustrate the present invention more specifically. Examples illustrating the preparation of starting materials or the use of the compounds of the present invention may be found in EP—A—48136.

Example 1

Sodium methoxide (1.134 g) was dissolved in 20 ml of anhydrous methanol, and the solution was stirred at room temperature in an atmosphere of argon. To the solution was added dropwise over 30 minutes at room temperature 20 ml of an anhydrous methanol solution of 2.692 g of alpha-bromo-p-isobutylpropiophenone. After the addition, 50 ml of water was added to the reaction mixture, and the mixture was extracted with 20 ml of diethyl ether three times. The extract was washed with 20 ml of water, dried over anhydrous magnesium sulfate and potassium carbonate, and concentrated under reduced pressure in the presence of a small amount of potassium carbonate to give 2.370 g of alpha-hydroxy-p-isobutyl-propiophenone dimethyl acetal as a colorless oily substance. The yield was 94%. For elemental analysis, the product was purified by column chromatography (Florisil; methylene chloride).

IR (neat): 3500, 2950, 1460, 1100, 1050, 980, 850, 800, 740 $cm^{-1}$.

NMR($CDCl_3$): δ 0.91 (6H, d, J=7Hz), 0.96 (3H, d, J=7Hz), 1.6—2.1 (1H, m), 2.43 (2H, d, J=7Hz), 2.57 (1H, broad s), 3.20 (3H, s), 3.30 (3H, s), 4.06 (1H, q, J=7Hz), 7.08 (2H, d, J=9Hz), 7.33 (2H, d, J=9Hz).

For $C_{15}H_{24}O_3$:

Calculated: C, 71.39; H, 9.59%.

Found: C, 71.39; H, 9.48%.

Example 2

Sodium methoxide (0.979 g) was dissolved in 10 ml of anhydrous methanol, and the solution was stirred at room temperature in an argon atmosphere. To the solution was added dropwise over 20 minutes 10 ml of an anhydrous methanol solution of 2.036 g of alpha-chloro-p-isobutylpropiophenone and the mixture was further stirred at room temperature for 4 hours. Work-up gave 2.158 g (yield 95%) of alpha-hydroxy-p-isobutylpropiophenone dimethyl acetal as a pale yellow oil. Yield 95%.

Example 3

Sodium methoxide (9.20 g) was suspended in 10 ml of anhydrous diethyl ether, and in an atmosphere of argon, the solution was stirred under ice cooling. To the solution was added dropwise over 40 minutes 80 ml of an anhydrous diethyl ether solution of 15.0 g of alpha-bromo-p-methoxypropiophenone. After the addition, the insoluble matter was separated by filtration in a stream of argon. The filtrate was concentrated under reduced pressure to give 7.88 g of 1-(4-methoxyphenyl)-1-methoxy-1,2-epoxypropane as a pale red oil.

Yield: 68%

NMR($CDCl_3$): δ 1.00 (3H, d, J=6Hz), 3.23 (3H, s), 3.52 (1H, q, J=6Hz), 3.82 (3H, s), 6.92 (2H, d, J=7Hz), 7.38 (2H, d, J=7Hz).

Example 4

7.88 g of 1-(4-methoxyphenyl)-1-methoxy-1,2-epoxypropane and 200 mg of sodium methoxide were stirred in 30 ml of anhydrous methanol at room temperature for 15 hours. Water (50 ml) was added to the reaction solution, and the mixture was extracted with 20 ml of diethyl ether three times. The extracts were dried over anhydrous magnesium sulfate and anhydrous potassium carbonate, and concentrated under reduced pressure in the presence of a small amount of anhydrous potassium carbonate to give 8.26 g of alpha-hydroxy-p-methoxy-propiophenone dimethyl acetal as a colorless oil. For elemental analysis, this product was purified by column chromatography (Florisil; methylene chloride).

Yield: 61%

IR (neat): 3520, 1050 $cm^{-1}$.

NMR($CDCl_3$): δ 0.94 (3H, d, J=7Hz), 2.35 (1H, d, J=4Hz), 3.20 (3H, s), 3.33 (3H, s), 3.78 (3H, s), 4.05 (1H, dq, J=4 and 7Hz), 6.18 (2H, d, J=9Hz), 7.35 (2H, d, J=9Hz).

For $C_{12}H_{18}O_4$:

Calculated: C, 63.70; H, 8.02%.

Found: C, 63.98; H, 8.08%.

Example 5

Sodium methoxide (1.62 g) was dissolved in 20 ml of anhydrous methanol, and the solution was stirred at room temperature under an atmosphere of argon. To the resulting solution was added 20 ml of an anhydrous methanol solution of 2.64 g of alpha-bromo-p-(tert-butyl)isovalerophenone over 15 minutes and the mixture was stirred at room temperature for 18 hours.

Usual work-up as in Example 1 gave 2.44 g of alpha-hydroxy-p-(tert-butyl)isovalerophenone dimethyl acetal as a pale yellow oil.

Yield: 98%

IR (neat): 2950, 1110, 1040 $cm^{-1}$.

NMR($CDCl_3$): δ 0.70 (3H, d, J=7Hz), 0.90 (3H, d, J=7Hz), 1.32 (9H, s), 1.0—1.6 (1H, m), 2.45 (1H, broad s), 3.22 (3H, s), 3.25 (3H, s), 3.67 (1H, d, J=6Hz), 7.33 (4H, s).

Example 6

Metallic sodium (0.46 g) was dissolved in 10 ml of anhydrous methanol, and the solution was stirred at room temperature. To the solution was added dropwise over 10 minutes 10 ml of an anhydrous methanol solution of 2.19 g of 1-(2-thienyl)-2-bromo-1-propanone and the mixture was stirred further for 3.5 hours at room temperature. Usual work-up as in Example 1 gave 1.900 g of 1-(2-thienyl)-2-hydroxy-1-propanone dimethyl acetal as a colorless oil.

Yield: 94%

IR (neat): 3450, 1110, 1055, 980, 855, 710 $cm^{-1}$.

NMR($CDCl_3$): δ 1.05 (3H, d, J=7Hz), 2.54 (1H, broad s), 3.26 (3H, s), 3.30 (3H, s), 4.16 (1H, broad q, J=7Hz), 6.9—7.2 (2H, m), 7.2—7.4 (1H, m).

Example 7

By a similar operation to Example 6, 1-(4-methoxyphenyl)-2-hydroxy-1-propanone dimethyl acetal was prepared as a pale yellow oil from 1-(4-methoxyphenyl)-2-bromo-1-propanone.

Example 8

Metallic sodium (1.30 g, 56.5 millimoles) was dissolved in 30 ml of anhydrous ethanol, and the mixture was stirred at room temperature. To the solution was added dropwise over 15 minutes 20 ml of an anhydrous ethanol solution of 5.33 g (0.025 mole) of alpha-bromopropiophenone, and the mixture was stirred further for 40 minutes at room temperature. Water (100 ml) was added, and the mixture was extracted with 60 ml of diethyl ether twice. The extracts were washed with water, dried over anhydrous magnesium sulfate and anhydrous potassium carbonate, and concentrated under reduced pressure. The residue was dissolved in 30 ml of anhydrous ethanol containing a catalytic amount of metallic sodium dissolved therein, and the solution was stirred for 2 days at room temperature. Usual work-up as in Example 1 gave 4.497 g of 1-phenyl-2-hydroxy-1-propanone diethyl acetal as a colorless oil.

Yield: 80.2%

IR (neat): 2960, 1452, 1120, 1092, 1057, 1040, 772, 709 $cm^{-1}$.

NMR($CDCl_3$): δ 0.94 (3H, d, J=6Hz), 1.05—1.40 (6H, m), 2.61 (1H, broad s), 3.22—3.83 (4H, m), 4.05 (1H, q, J=6Hz), 7.1—7.7 (5H, m).

Example 9

By a similar operation to Example 6 1-(4-acetylaminophenyl)-2-hydroxy-1-propanone dimethyl acetal was prepared as a glassy substance from 1-(4-acetylaminophenyl)-2-chloro-1-propanone.

NMR($CDCl_3$): δ 0.95 (3H, d, J=7Hz), 2.14 (3H, s), 2.94 (1H, broad s), 3.21 (3H, s), 3.33 (3H, s), 4.10 (1H, q, J=7Hz), 7.37 (2H, d, J=8Hz), 7.51 (2H, d, J=8Hz), 8.93 (1H, broad s).

Example 10

In the same way as in Example 6, 1-(4-fluorophenyl)-2-hydroxy-1-propanone dimethyl acetal was prepared as a pale yellow oil from 1-(4-fluorophenyl)-2-bromo-1-propanone.

IR (neat): 2975, 2937, 1610, 1512, 1230, 1159, 1105, 1053, 982, 839 $cm^{-1}$.

NMR($CDCl_3$): δ 0.93 (3H, d, J=6Hz), 2.53 (1H, broad s), 3.23 (3H, s), 3.33 (3H, s), 4.08 (1H, broad q, J=6Hz), 6.83—7.18 (2H, m), 7.32—7.60 (2H, m).

Example 11

In a similar operation to Example 1, 1-(6-methoxy-2-naphthyl)-2-hydroxy-1-propanone dimethyl acetal was prepared as a colorless oil from 1-(6-methoxy-2-naphthyl)-2-bromo-1-propanone. Yield, 100%. The product crystallized after it was purified by column chromatography (Florisil, methylene chloride) and allowed to stand at room temperature.

Colorless crystals.

m.p.: 56—59°C.

IR (KBr): 3500, 1637, 1610, 1488, 1276, 1215, 1175, 1118, 1106, 1040, 859 $cm^{-1}$.

NMR($CDCl_3$): δ 0.97 (3H, d, J=7Hz), 2.48 (1H, broad s), 3.20 (3H, s), 3.36 (3H, s), 3.82 (3H, s), 4.14 (1H, q, J=7Hz), 7.00— 7.24 (2H, m), 7.40—7.98 (4H, m).

For $C_{16}H_{20}O_4$:

Calculated: C, 69.54; H, 7.30%
Found: C, 69.25; H, 7.28%

Example 12

350 mg of sodium metal was dissolved in 40 ml of anhydrous methanol and the solution was stirred at room temperature. 2.93 g of 1-(6-methoxy-2-naphthyl)-2-bromo-1-propanone was added to the solution, followed by stirring for 24 hours. 50 ml of water was added to the mixture which was then extracted with methylene chloride (15 ml × 4). The extract was dried over anhydrous magnesium sulfate and anhydrous potassium carbonate and concentrated under reduced pressure to obtain 2.845 g of a crude product of 1-(6-methoxy-2-naphthyl)-2-hydroxy-1-propanone dimethyl acetal.

Example 13

By a similar operation to Example 6, 1-(4-chlorophenyl)-2-hydroxy-1-propanone dimethyl acetal was prepared as a colorless oil from 1-(4-chlorophenyl)-2-bromo-1-propanone.

IR (neat): 3450, 2975, 2930, 1600, 1492, 1398, 1108, 1095, 1052, 1016, 980 829, 743 $cm^{-1}$.

Example 14

In the same way as in Example 6, 1-[4-(1-oxo-2-isoindolinyl)phenyl]-2-hydroxy-1-propanone dimethyl acetal was obtained from 1-[4-(1-oxo-2-isoindolinyl)phenyl]-2-chloro-1-propanone in a yield of 83% as colorless crystals having a melting point of 130 to 135°C.

IR (KBr): 3530, 1680, 1515, 1385, 1310, 1120, 1045, 740 $cm^{-1}$.

NMR($CDCl_3$): δ 0.96 (3H, d, J=7Hz), 2.36 (1H, broad s), 3.20 (3H, s), 3.33 (3H, s), 4.10 (1H, broad q, J=7Hz), 4.80 (2H, s), 7.4—7.6 (5H, m), 7.8—8.0 (3H, m).

For $C_{19}H_{21}NO_4$:

Calculated: C, 69.70; H, 6.47; N, 4.28%
Found: C, 69.63; H, 6.49; N, 4.21%

Example 15

By the same operation as in Example 6, 1-(4-biphenylyl)-2-hydroxy-1-propanone dimethyl acetal was prepared as colorless crystals having a melting point of 78.5 to 80°C in a yield of 95% from 1-(4-biphenylyl)-2-bromo-1-propanone.

m.p.: 78.5—80°C (from ether/n-hexane)

NMR($CDCl_3$): δ 1.00 (3H, d, J=7Hz), 2.44 (1H, d, J=3.1Hz), 3.23 (3H, s), 3.37 (3H, s), 4.12 (1H, q, d, J=7Hz, 3.1Hz), 7.2—7.7 and 7.55 (m and s, 9H).

IR (KBr): 1118, 1102, 1045, 1007, 979, 838, 770, 735, 695 $cm^{-1}$.

For $C_{17}H_{20}O_3$:

Calculated: C, 74.97; H, 7.40%
Found: C, 74.88; H, 7.27%

Example 16

By a similar operation as in Example 6, 1,1-dimethoxy-2-hydroxy-1,2,3,4-tetrahydronaphthalene was prepared from 2-bromo-1-oxo-1,2,3,4-tetrahydronaphthalene.

IR (neat): 1138, 1080, 1058, 767 $cm^{-1}$.

Example 17

By a similar operation to Example 6, 1-(4-difluoromethoxyphenyl)-2-hydroxy-1-propanone dimethyl acetal was prepared as a colorless oil from 2-bromo-1-(4-difluoromethoxyphenyl)-propanone.

IR (neat): 3600—3200, 1515, 1385, 1230, 1130, 1050, 840 $cm^{-1}$.

NMR($CDCl_3$): δ 0.95 (3H, d, J=Hz), 2.37 (1H, d, J=3Hz), 3.23 (3H, s), 3.33 (3H, s), 4.10 (1H, dq, J=3 and 7Hz), 6.52 (1H, t, J=74Hz), 7.10 (2H, d, J=9Hz), 7.49 (2H, d, J=9Hz).

Example 18

By a similar operation to Example 6, 1-(4-difluoromethoxyphenyl)-2-hydroxy-3-methyl-1-butanone dimethyl acetal was prepared as a colorless oil from 2-bromo-1-(4-difluoromethoxyphenyl)-3-methyl-1-butanone.

IR (neat): 3600—3300, 1515, 1390, 1230, 1130, 1050 $cm^{-1}$.

NMR($CDCl_3$): δ 0.69 (3H, d, J=6Hz), 0.88 (3H, d, J=6Hz), 1.2—1.7 (1H, m), 2.50 (1H, d, J=3Hz), 3.22 (3H, s), 3.24 (3H, s), 3.70 (1H, dq, J=3 and 6Hz), 6.50 (1H, t, J=74Hz), 7.07 (2H, d, J=9Hz), 7.52 (2H, d, J=9Hz).

Example 19

230 mg of sodium metal was dissolved in 3 ml of anhydrous methanol. To the solution was added an anhydrous methanol solution (2 ml) of 1.43 g of 2-bromo-1-(4-ethoxyphenyl)-3-methyl-1-butanone. The mixture was stirred at room temperature for 1.5 hours, then at 50°C for 45 minutes. Usual work-up gave 1.344 g of a crude product of 1-(4-ethoxyphenyl)-2-hydroxy-3-methyl-1-butanone dimethyl acetal as a colorless without further purification, oily substance.

IR (neat): 3400—3300, 1610, 1515, 1485, 1400, 1250, 1175, 1115, 1050, 990 925, 840, 810 $cm^{-1}$.

NMR ($CDCl_3$): γ 0.68 (3H, t, J=6Hz), 0.87 (3H, d, J=6Hz), 1.39 (3H, t, J=7Hz), 1.2—1.7 (1H, m), 2.50 (1H, d, J=3Hz), 3.22 (3H, s), 3.24 (3H, s), 3.68 (1H, dd, J=3 and 5Hz), 4.01 (2H, q, J=7Hz), 6.82 (2H, d, J=9Hz), 7.37 (2H, d, J=9Hz).

Example 20

In a similar way as in Example 19, 2.58 g of the crude product of 1-(4-methoxyphenyl)-2-hydroxy-3-methyl-1-butanone dimethyl acetal was prepared as a colorless oil from 2.71 g of 2-bromo-1-(4-methoxyphenyl)-3-methyl-1-butanone without purification.

IR (neat): 3600—3300, 1615, 1515, 1255 1175, 1115, 1045, 840 $cm^{-1}$.

NMR($CDCl_3$): δ 0.70 (3H, d, J=6Hz), 0.87 (3H, d, J=6Hz), 1.2—1.7 (1H. m), 2.53 (1H, broad s), 3.33 (3H, s), 3.34 (3H, s), 3.69 (1H, d, J=5Hz), 3.78 (3H, s), 6.83 (2H, d, J=9Hz), 7.40 (2H, d, J=9Hz).

Example 21

To a stirred suspension of 0.65 g of sodium hydride (55% in mineral oil) in anhydrous tetrahydrofuran (10 ml) was added 2.7 ml of ethylene glycol under ice-cooling. After stirring for 40 minutes at room temperature, 2.71 g of 2-bromo-1-(4-methoxyphenyl)-3-methyl-1-butanone was added to the reaction mixture and the mixture was stirred for 12 hours at 60°C. After addition of water (50 ml), the reaction mixture was extracted with 30 ml of methylene chloride three times. The extracts were washed with 10 ml of water, dried over magnesium sulfate, and concentrated in vacuo. The oily residue was subjected to column chromatography (Florisil, methylene chloride) to yield 719 mg of 2-hydroxy-1-(4-methoxyphenyl)-3-methyl-1-butanone ethylene acetal as a colorless oil. Yield 29%

IR (neat): 3600—3400, 1620, 1520, 1255, 1175, 1040, 845 $cm^{-1}$.

NMR($CDCl_3$): δ 0.88 (3H, d, J=6Hz), 0.90 (3H, d, J=6Hz), 1.2—1.7 (1H. m), 2.55 (1H, broad d, J=5Hz), 3.5—4.2 (5H, m), 3.77 (3H, s), 6.83 (2H, d, J=9Hz), 7.36 (2H, d, J=9Hz).

**Claims for the Contracting States: BE CH DE FR GB IT LI LU NL SE**

1. An alpha-hydroxyketone acetal of the formula (VI):

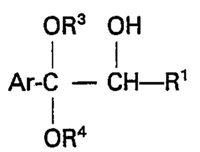

(VI)

wherein Ar represents an aromatic group, $R^1$ represents a hydrogen atom or a saturated aliphatic group, or Ar and $R^1$ form a condensed ring together with the carbon atom to which they are bonded, and $R^3$ and $R^4$ independently represent alkyl groups or together represent an alkylene group, subject to the provisos that:

(i) Ar does not represent phenyl or 4-chlorophenyl when $R^1$ is a hydrogen atom;

(ii) Ar does not represent phenyl when all of $R^1$, $R^3$ and $R^5$ are methyl;

(iii) Ar does not represent an unsubstituted pyridyl group when $R^3$ and $R^4$ together represent an ethylene group and $R^4$ represents a hydrogen atom;

(iv) Ar does not represent a p-(4,4-dimethyl-oxazolin-2-yl)-phenyl group when $R^3$ and $R^4$ together represent an ethylene group and $R^1$ represents an isopropyl group; and

(v) Ar does not represent 1-methyl-3-indolyl when $R^3$ and $R^4$ together represent an ethylene group and $R^1$ represents a hydrogen atom or a methyl group.

2. An acetal according to claim 1 wherein Ar is an aromatic hydrocarbon group, optionally having one or more substituent on an aromatic ring, or a heteroaromatic group containing at least one nitrogen, oxygen or sulfur heteroatom, optionally having one or more substituent on a heteroaromatic ring.

3. An acetal according to claim 2 wherein Ar represents a 6-methoxy-2-naphthyl group, a 4-isobutylphenyl group, a 4-lower alkoxyphenyl group, a 4-difluoromethoxyphenyl group, a 2-thienyl group, a 4-(1-oxo-2-isoindolinyl) phenyl group, a 4-biphenylyl group, or a 4-(tert-butyl) phenyl group.

4. An acetal according to claim 1, 2 or 3 wherein $R^1$ represents a linear, branched or cyclic alkyl group of up to 10 carbon atoms.

5. An acetal according to claim 4 wherein $R^1$ represents a linear or branched alkyl group of 1 to 6 carbon atoms or a cycloalkyl group of 3 to 7 carbon atoms.

6. An acetal according to any one of the preceding claims wherein $R^3$ and $R^4$ independently represent alkyl groups of 1 to 4 carbon atoms, or together represent an alkylene group of up to 4 carbon atoms.

7. An acetal according to claim 1 which is 1-(6-methoxy-2-naphthyl)-2-hydroxy-1-propanone dimethyl acetal.

8. A process for preparing a compound as defined in claim 1 wherein $R^3$ is identical to $R^4$ which comprises reacting a compound of general formula (V)

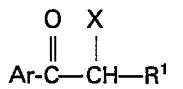

(V)

wherein Ar and $R^1$ are as defined in claim 1 and X represents a halogen atom, with an alkali metal alkoxide of formula $R^3OM$ in the presence of the corresponding alcohol of formula $R^3OH$ wherein $R^3$ is as defined in claim 1 and M represents an alkali metal atom.

9. A process for preparing a compound as defined in claim 1, which comprises reacting a compound of general formula (V)

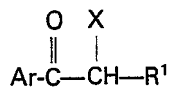

(V)

wherein Ar and $R^1$ are as defined in claim 1 and X represents a halogen atom with an alkali metal alkoxide of formula $R^3OM$ wherein $R^3$ is as defined in claim 1 and M represents an alkali metal atom in an aprotic solvent to form an epoxy compound of formula (VII)

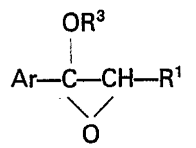

(VII)

and then reacting the epoxy compound of formula (VII) with an alcohol of formula $R^4OH$ in the presence of a catalytic amount of an alkali metal alkoxide of formula $R^4OM$ wherein $R^4$ is as defined in claim 1 and M represents an alkali metal atom.

**Claims for the Contracting State: AT**

1. A process for preparing an alpha-hydroxyketone acetal of the formula (VI):

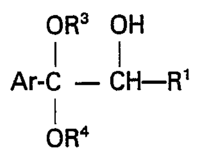

(VI)

wherein Ar represents an aromatic group, $R^1$ represents a hydrogen atom or a saturated aliphatic group, or Ar and $R^1$ form a condensed ring together with the carbon atom to which they are bonded, and $R^3$ and $R^4$ independently represent alkyl groups or together represent an alkylene group, subject to the provisos that:

(i) Ar does not represent phenyl or 4-chlorophenyl when $R^1$ is a hydrogen atom;

(ii) Ar does not represent phenyl when all of $R^1$, $R^3$ and $R^4$ are methyl;

(iii) Ar does not represent an unsubstituted pyridyl group when $R^3$ and $R^4$ together represent an ethylene group and $R^1$ represents a hydrogen atom;

(iv) Ar does not represent a p-(4,4-dimethyl-oxazolin-2-yl)-phenyl group when $R^3$ and $R^4$ together represent an ethylene group and $R^1$ represents an isopropyl group; and

(v) Ar does not represent 1-methyl-3-indolyl when $R^3$ and $R^4$ together represent an ethylene group and $R^1$ represents a hydrogen atom or a methyl group, which comprises reacting a compound of general formula (V)

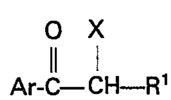

(V)

wherein Ar and $R^1$ are as defined above and X represents a halogen atom, with an alkali metal alkoxide of formula $R^3OM$ in the presence of the corresponding alcohol of formula $R^3OH$ wherein $R^3$ is as defined above and M represents an alkali metal atom.

2. A process for preparing a compound as defined in claim 1, which comprises reacting a compound of general formula (V)

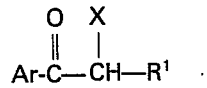

(V)

wherein Ar and $R^1$ are as defined in claim 1 and X represents a halogen atom with an alkali metal alkoxide of formula $R^3OM$ wherein $R^3$ is as defined in claim 1 and M represents an alkali metal atom in an aprotic solvent to form an epoxy compound of formula (VII)

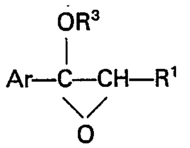

(VII)

and then reacting the epoxy compound of formula (VII) with an alcohol of formula $R^4OH$ in the presence of a catalytic amount of an alkali metal alkoxide of formula $R^4OM$ wherein $R^4$ is as defined in claim 1 and M represents an alkali metal atom.

3. A process according to claim 1 or 2 wherein Ar is an aromatic hydrocarbon group, optionally having one or more substituent on an aromatic ring, or a heteroaromatic group containing at least one nitrogen, oxygen or sulfur heteroatom, optionally having one or more substituent on a heteroaromatic ring.

4. A process according to claim 3 wherein Ar represents a 6-methoxy-2-naphthyl group, a 4-isobutylphenyl group, a 4-lower alkoxyphenyl group, a 4-difluoromethoxyphenyl group, a 2-thienyl group, a 4-(1-oxo-2-isoindolinyl)phenyl group, a 4-biphenylyl group, or a 4-(tert-butyl)phenyl group.

5. A process according to any one of the preceding claims wherein $R^1$ represents a linear, branched or cyclic alkyl group of up to 10 carbon atoms.

6. A process according to claim 5 wherein $R^1$ represents a linear or branched alkyl group of 1 to 6 carbon atoms or a cycloalkyl group of 3 to 7 carbon atoms.

7. A process according to any one of the preceding claims wherein $R^3$ and $R^4$ independently represent alkyl groups of 1 to 4 carbon atoms, or together represent an alkylene group of up to 4 carbon atoms.

8. A process according to claim 1 or 2 wherein the acetal is 1-(6-methoxy-2-naphthyl)-2-hydroxy-1-propanone dimethyl acetal.

**Patentansprüche für die Vertragsstaaten: BE CH DE FR GB IT LI LU NL SE**

1. Alpha-hydroxyketon-acetal der Formel (VI):

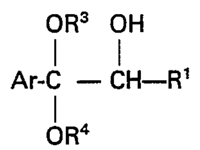

(VI)

in welcher Ar für eine aromatische Gruppe steht, $R^1$ ein Wasserstoffatom oder eine gesättigte aliphatische Gruppe darstellt, oder Ar und $R^1$ zusammen mit dem Kohlenstoffatom an das sie gebunden sind, einen kondensierten Ring bilden, und $R^3$ und $R^4$ unabhängig voneinander Alkylgruppen repräsentieren oder zusammen für eine Alkylengruppe stehen, unter den Voraussetzungen, daß:

(i) Ar nicht Phenyl oder 4-Chlorphenyl bedeutet, wenn $R^1$ ein Wasserstoffatom ist;

(ii) Ar nicht Phenyl bedeutet, wenn alle $R^1$, $R^3$ und $R^4$ für Methyl stehen;

(iii) Ar keine unsubstituierte Pyridylgruppe bedeutet, wenn $R^3$ und $R^4$ zusammen für eine Ethylengruppe stehen und $R^1$ ein Wasserstoffatom repräsentiert;

(iv) Ar nicht eine p-(4,4-Dimethyl-oxazolin-2-yl)-phenylgruppe bedeutet, wenn $R^3$ und $R^4$ zusammen eine Ethylengruppe darstellen und $R^1$ eine Isopropylgruppe repräsentiert; und

(v) Ar nicht 1-Methyl-3-indolyl bedeutet, wenn $R^3$ und $R^4$ zusammen eine Ethylengruppe darstellen und $R^1$ ein Wasserstoffatom oder eine Methylgruppe repräsentiert.

2. Acetal nach Anspruch 1, in welchem Ar eine aromatische Kohlenwasserstoffgruppe, die gegebenenfalls einen oder mehrere Substituenten an einem aromatischen Ring aufweist, oder eine heteroaromatische Gruppe, die wenigstens ein Stickstoff-, Sauerstoff- oder Schwefelheteroatom enthält, gegebenenfalls mit einem oder mehreren Substituenten an einem heteroaromatischen Ring, darstellt.

3. Acetal nach Anspruch 2, in welchem Ar für eine 6-Methoxy-2-naphthylgruppe, eine 4-Isobutylphenylgruppe, eine 4-Niederalkoxyphenylgruppe, eine 4-Difluormethoxyphenylgruppe, eine 2-Thienylgruppe, eine 4-(1-Oxo-2-isoindolinyl)phenylgruppe, eine 4-Biphenylylgruppe oder eine 4-(tert-Butyl)phenylgruppe steht.

4. Acetal nach Anspruch 1, 2 oder 3, in welchem $R^1$ eine lineare, verzweigte oder cyclische Alkylgruppe mit bis zu 10 Kohlenstoffatomen darstellt.

5. Acetal nach Anspruch 4, in welchem $R^1$ eine lineare oder verzweigte, Alkylgruppe mit 1 bis 6 Kohlenstoffatomen oder eine Cycloalkylgruppe mit 3 bis 7 Kohlenstoffatomen darstellt.

6. Acetal nach einem der vorangehenden Ansprüche, in welchen $R^3$ und $R^4$ unabhängig voneinander Alkylgruppen mit 1 bis 4 Kohlenstoffatomen repräsentieren oder zusammen eine Alkylengruppe mit bis zu 4 Kohlenstoffatomen darstellen.

7. Acetal nach Anspruch 1, nämlich 1-(6-Methoxy-2-naphthyl)-2-hydroxy-1-propanon-dimethylacetal.

8. Verfahren zur Herstellung einer Verbindung wie in Anspruch 1 definiert, bei welchem $R^3$ und $R^4$ identisch sind, welches umfaßt die Umsetzung einer Verbindung der allgemeinen Formel (V)

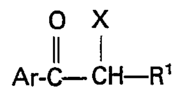

(V)

in welcher Ar und $R^1$ wie in Anspruch 1 definiert sind und X ein Halogenatom bedeutet, mit einem Alkalimetallalkoxid der Formel $R^3OM$ in der Anwesenheit des entsprechenden Alkohols der Formel $R^3OH$, in welchem $R^3$ wie in Anspruch 1 definiert ist und M für eine Alkalimetallatom steht.

9. Verfahren zur Herstellung einer Verbindung wie in Asnpruch 1 definiert, welches umfaßt die Umsetzung einer Verbindung der allgemeinen Formel (V)

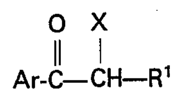

(V)

in welcher Ar und $R^1$ wie in Anspruch 1 definiert sind und X für ein Halogenatom steht, mit einem Alkalimetallalkoxid der Formel $R^3OM$, in welcher $R^3$ wie in Anspruch 1 definiert ist und M ein Alkalimetallatom darstellt, in einem aprotischen Lösungsmittel unter Bildung einer Epoxyverbindung der Formel (VII)

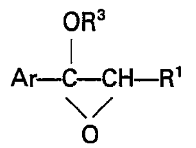

(VII)

und anschließende Umsetzung der Epoxyverbindung der Formel (VII) mit einem Alkohol der Formel $R^4OH$ in Anwesenheit einer katalytischen Menge eines Alkalimetallalkoxids der Formel $R^4OM$, in welcher $R^4$ wie in Anspruch 1 definiert ist und M ein Alkalimetallatom darstellt.

**Patentansprüche für den Vertragsstaat: AT**

1. Verfahren zur Herstellung eines alpha-Hydroxyketonacetals der Formel (VI):

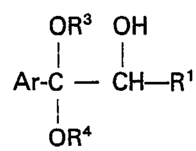

(VI)

in welcher Ar für eine aromatische Gruppe steht, $R^1$ ein Wasserstoffatom oder eine gesättigte aliphatische Gruppe darstellt, oder Ar und $R^1$ zusammen mit dem Kohlenstoffatom an das sie gebunden sind, einen kondensierten Ring bilden, und $R^3$ und $R^4$ unabhängig voneinander Alkylgruppen repräsentieren oder zusammen für eine Alkylengruppe stehen, unter den Voraussetzungen, daß:

(i) Ar nicht Phenyl oder 4-Chlorphenyl bedeutet, wenn $R^1$ ein Wasserstroffatom ist;

(ii) Ar nicht Phenyl bedeutet, wenn alle $R^1$, $R^3$ und $R^4$ für Methyl stehen;

(iii) Ar keine unsubstituierte Pyridylgruppe bedeutet, wenn $R^3$ und $R^4$ zusammen für eine Ethylengruppe stehen und $R^1$ ein Wasserstoffatom repräsentiert;

(iv) Ar nicht eine p-(4,4-Dimethyl-oxazolin-2-yl)-phenylgruppe bedeutet, wenn $R^3$ und $R^4$ zusammen eine Ethylengruppe darstellen und $R^1$ eine Isopropylgruppe repräsentiert; und

(v) Ar nicht 1-Methyl-3-indolyl bedeutet, wenn $R^3$ und $R^4$ zusammen eine Ethylengruppe darstellen und $R^1$ ein Wasserstoffatom oder eine Methylgruppe repräsentiert, welches umfaßt die Umsetzung einer Verbindung der allgemeinen Formel (V)

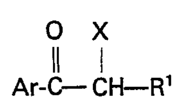

(V)

in welcher Ar und $R^1$ wie oben definiert sind und X ein Halogenatom bedeutet, mit einem Alkalimetallalkoxid der Formel $R^3OM$ in der Anwesenheit des entsprechenden Alkohols der Formel $R^3OH$, in welchem $R^3$ wie oben definiert ist und M für eine Alkalimetallatom steht.

2. Verfahren zur Herstellung einer Verbindung wie in Anspruch 1 definiert, welches umfaßt die Umsetzung einer Verbindung der allgemeinen Formel (V)

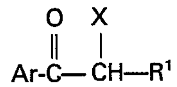

(V)

in welcher Ar und $R^1$ wie in Anspruch 1 definiert sind und X für ein Halogenatom steht, mit einem Alkalimetallalkoxid der Formel $R^3OM$, in welcher $R^3$ wie in Anspruch 1 definiert ist und M ein Alkalimetallatom darstellt, in einem aprotischen Lösungsmittel unter Bildung einer Epoxyverbindung der Formel (VII)

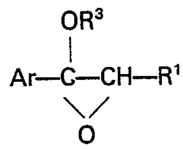

(VII)

und anschließende Umsetzung der Epoxyverbindung der Formel (VII) mit einem Alkohol der Formel $R^4OH$ in Anwesenheit einer katalytischen Menge eines Alkalimetallalkoxids der Formel $R^4OM$, in welcher $R^4$ wie in Anspruch 1 definiert ist und M ein Alkalimetallatom darstellt.

3. Verfahren nach Anspruch 1 oder 2, bei welchem Ar eine aromatische Kohlenwasserstoffgruppe, die gegebenenfalls einen oder mehrere Substituenten an einem aromatischen Ring aufweist, oder eine heteroaromatische Gruppe, die wenigstens ein Stickstoff-, Sauerstoff- oder Schwefelheteroatom enthält, gegebenenfalls mit einem oder mehreren Substituenten an einem heteroaromatischen Ring, darstellt.

4. Verfahren nach Anspruch 3, bei welchem Ar für eine 6-Methoxy-2-naphthylgruppe, eine 4-Isobutylphenylgruppe, eine 4-Niederalkoxyphenylgruppe, eine 4-Difluormethoxyphenylgruppe, eine 2-Thienylgruppe, eine 4-(1-Oxo-2-isoindolinyl)phenylgruppe, eine 4-Biphenylylgruppe oder eine 4-(tert-Butyl)phenylgruppe steht.

5. Verfahren nach einem der vorangehenden Ansprüche, bei welchem $R^1$ eine lineare, verzweigte oder cyclische Alkylgruppe mit bis zu 10 Kohlenstoffatomen darstellt.

6. Verfahren nach Anspruch 5, bei welchem $R^1$ eine lineare oder verzweigte Alkylgruppe mit 1 bis 6 Kohlenstoffatomen oder eine Cycloalkylgruppe mit 3 bis 7 Kohlenstoffatomen darstellt.

7. Verfahren nach einem der vorangehenden Ansprüche, bei welchem $R^3$ und $R^4$ unabhängig voneinander Alkylgruppen mit 1 bis 4 Kohlenstoffatomen repräsentieren oder zusammen eine Alkylengruppe mit bis zu 4 Kohlenstoffatomen darstellen.

8. Verfahren nach Anspruch 1 oder 2 bei welchem das Acetal 1-(6-Methoxy-2-naphthyl)-2-hydroxy-1-propanondimethylacetal ist.

**Revendications pour les Etats contractants: BE CH DE FR GB IT LI LU NL SE**

1. Acétal d'alpha-hydroxycétone de formule générale (VI):

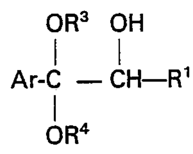

(VI)

dans laquelle Ar représente un groupe aromatique, $R^1$ représente un atome d'hydrogène ou un groupe aliphatique saturé, ou bien Ar et $R^1$ forment un noyau condensé conjointement avec l'atome de carbone auquel ils sont liés, et $R^3$ et $R^4$ représentent, indépendamment, des groupes alkyle ou représentent conjointement un groupe alkylène, sous réserve que:

(i) Ar ne représente pas un groupe phényle ou 4-chlorophényle lorsque $R^1$ est un atome d'hydrogène;

(ii) Ar ne représente pas un groupe phényle lorsque $R^1$, $R^3$ et $R^4$ représentent tous des groupes méthyle;

(iii) Ar ne représente pas un groupe pyridyle non substitué lorsque $R^3$ et $R^4$ représentent conjointement un groupe éthylène et $R^1$ représente un atome d'hydrogène;

(iv) Ar ne représente pas un groupe p-(4,4-diméthyloxazoline-2-yle)-phényle lorsque $R^3$ et $R^4$ représentent conjointement un groupe éthylène et $R^1$ représente un groupe isopropyle; et

(v) Ar ne représente pas un groupe 1-méthyl-3-indolyle lorsque $R^3$ et $R^4$ représentent conjointement un groupe éthylène et $R^1$ représente un atome d'hydrogène ou un groupe méthyle.

2. Acétal suivant la revendication 1, dans lequel Ar est un groupe hydrocarboné aromatique, portant

facultativement un ou plusieurs substituants sur un noyau aromatique, ou un groupe hétéroaromatique contenant au moins un hétéroatome d'azote, d'oxygène ou de soufre, portant facultativement un ou plusieurs substituants sur un noyau hétéroaromatique.

3. Acétal suivant la revendication 2, dans lequel Ar représente un groupe 6-méthoxy-2-naphtyle, un groupe 4-isobutylphényle, un groupe 4-(alkoxy inférieur)phényle, un groupe 4-difluorométhoxyphényle, un groupe 2-thiényle, un groupe 4-(1-oxo-2-isoindolinyl)phényle, un groupe 4-biphénylyle ou un groupe 4-(tertiobutyl)phényle.

4. Acétal suivant la revendication 1, 2 ou 3, dans lequel $R^1$ représente un groupe alkyle linéaire, ramifié ou cyclique ayant jusqu'à 10 atomes de carbone.

5. Acétal suivant la revendication 4, dans lequel $R^1$ représente un groupe alkyle linéaire ou ramifié de 1 à 6 atomes de carbone ou un groupe cycloalkyle de 3 à 7 atomes de carbone.

6. Acétal suivant l'une quelconque des revendications précédentes, dans lequel $R^3$ et $R^4$ représentent indépendamment des groupes alkyle ayant 1 à 4 atomes de carbone ou représentent conjointement un groupe alkylène ayant jusqu'à 4 atomes de carbone.

7. Acétal suivant la revendication 1, qui est le diméthylacétal de la 1-(6-méthoxy-2-naphthyl)-2-hydroxy-1-propanone.

8. Procédé de préparation d'un composé tel que défini dans la revendication 1, dans lequel $R^3$ est identique à $R^4$, qui consiste à faire réagir un composé de formule générale (V)

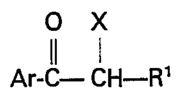
(V)

dans laquelle Ar et $R^1$ sont tels que définis dans la revendication 1 et X représente un atome d'halogène, avec un alcoolate de métal alcalin de formule $R^3OM$ en présence de l'alcool correspondant de formule $R^3OH$, où $R^3$ est tel que défini dans la revendication 1 et M représente un atome de métal alcalin.

9. Procédé de préparation d'un composé tel que défini dans la revendication 1, qui consiste à faire réagir un composé de formule générale (V)

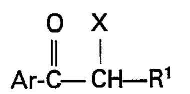
(V)

dans laquelle Ar et $R^1$ sont tels que définis dans la revendication 1 et X représente un atome d'halogène, avec un alcoolate de métal alcalin de formule $R^3OM$, dans laquelle $R^3$ est tel que défini dans la revendication 1 et M représente un atome de métal alcalin, dans un solvant aprotique pour former un composé époxy de formule (VII)

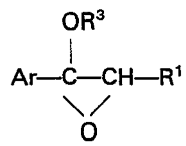
(VII)

puis à faire réagir le composé époxy de formule (VII) avec un alcool de formule $R^4OH$ en présence d'une quantité catalytique d'un alcoolate de métal alcalin de formule $R^4OM$, dans laquelle $R^4$ est tel que défini dans la revendication 1 et M représente un atome de métal alcalin.

**Revendications pour l'Etat contractant: AT**

1. Procédé de préparation d'un acétal d'alphahydroxycétone de formule (VI):

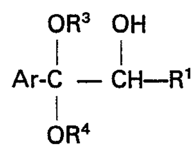
(VI)

dans laquelle Ar représente un groupe aromatique, $R^1$ représente un atome d'hydrogène ou un groupe aliphatique saturé, ou bien Ar et $R^1$ forment un noyau condensé conjointement avec l'atome de carbone auquel ils sont liés, et $R^3$ et $R^4$ représentent, indépendamment, des groupes alkyle ou représentent conjointement un groupe alkylène, sous réserve que:

(i) Ar ne représente pas un groupe phényle ou 4-chlorophényle lorsque $R^1$ est un atome d'hydrogène;

(ii) Ar ne représente pas un groupe phényle lorsque $R^1$, $R^3$ et $R^4$ représentent tous des groupes méthyle;

(iii) Ar ne représente pas un groupe pyridyle non substitué lorsque $R^3$ et $R^4$ représentent conjointement un groupe éthylène et $R^1$ représente un atome d'hydrogène;

(iv) Ar ne représente pas un groupe p-(4,4-diméthyloxazoline-2-yle)-phényle lorsque $R^3$ et $R^4$ représentent conjointement un groupe éthylène et $R^1$ représente un groupe isopropyle; et

(v) Ar ne représente pas un groupe 1-méthyl-3-indolyle lorsque $R^3$ et $R^4$ représentent conjointement un groupe éthylène et $R^1$ représente un atome d'hydrogène ou un groupe méthyle, qui consiste à faire réagir un composé de formule générale (V)

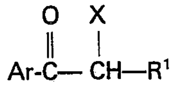

(V)

dans laquelle Ar et $R^1$ sont tels que définis ci-dessus et X représente un atome d'halogène, avec un alcoolate de métal alcalin de formule $R^3OM$ en présence de l'alcool correspondant de formule $R^3OH$ où $R^3$ est tel que défini ci-dessus et M représente un atome de métal alcalin.

2. Procédé de préparation d'un composé tel que défini dans la revendication 1, qui consiste à faire réagir un composé de formule générale (V)

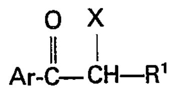

(V)

dans laquelle Ar et $R^1$ sont tels que définis dans la revendication 1 et X représente un atome d'halogène, avec un alcoolate de métal alcalin de formule $R^3OM$, dans laquelle $R^3$ est tel que défini dans la revendication 1 et M représente un atome de métal alcalin, dans un solvant aprotique pour former un composé époxy de formule (VII)

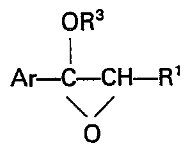

(VII)

puis à faire réagir le composé époxy de formule (VII) avec un alcool de formule $R^4OH$ en présence d'une quantité catalytique d'un alcoolate de métal alcalin de formule $R^4OM$, dans laquelle $R^4$ est tel que défini dans la revendication 1 et M représente un atome de métal alcalin.

3. Procédé suivant la revendication 1 ou 2, dans lequel Ar est un groupe hydrocarboné aromatique, portant facultativement un ou plusieurs substituants sur un noyau aromatique, ou un groupe hétéroaromatique contenant au moins un hétéroatome d'azote, d'oxygène ou de soufre, portant facultativement un ou plusieurs substituants sur un noyau hétéroaromatique.

4. Procédé suivant la revendication 3, dans lequel Ar représente un groupe 6-méthoxy-2-naphtyle, un groupe 4-isobutylphényle, un groupe 4-(alkoxy inférieur)-phényle, un groupe 4-difluorométhoxyphényle, un groupe 2-thiényle, un groupe 4-(1-oxo-2-isoindolinyl)-phényle, un groupe 4-biphénylyle ou un groupe 4-(tertiobutyl)-phényle.

5. Procédé suivant l'une quelconque des revendications précédentes, dans lequel $R^1$ représente un groupe alkyle linéaire, ramifié ou cyclique ayant jusqu'à 10 atomes de carbone.

6. Procédé suivant la revendication 5, dans lequel $R^1$ représente un groupe alkyle linéaire ou ramifié de 1 à 6 atomes de carbone ou un groupe cycloalkyle de 3 à 7 atomes de carbone.

7. Procédé suivant l'une quelconque des revendications précédentes, dans lequel $R^3$ et $R^4$ représentent indépendamment des groupes alkyle de 1 à 4 atomes de carbone, ou représentent conjointement un groupe alkylène ayant jusqu'à 4 atomes de carbone.

8. Procédé suivant la revendication 1 ou 2, dans lequel l'acétal est le diméthylacétal de la 1-(6-méthoxy-2-naphtyl)-2-hydroxy-1-propanone.